# EUROPÄISCHE PATENTSCHRIFT

(11) **EP 2 017 272 B1**
(45) Veröffentlichungstag und Bekanntmachung des Hinweises auf die Patenterteilung: **21.03.2012**
(21) Anmeldenummer: 08156112.8
(22) Anmeldetag: 13.05.2008
(51) Int. Cl.: C07D 311/32, C07D 311/40

(54) **Verfahren zum Freisetzen bestimmter Flavanone und Dihydrochalkone durch saure Hydrolyse**
Method to release certain flavanons and dihydrochalcones using acid hydrolysis
Procédé de libération de flavanones et de dihydrochalcones précis par hydrolyse acide

(30) Priorität: 10.05.2007 US 917100 P
(43) Veröffentlichungstag der Anmeldung: 21.01.2009
(73) Patentinhaber: Symrise AG, 37603 Holzminden (DE)
(72) Erfinder: Hilmer, Jens-Michael, 37603, Holzminden (DE); Ley, Jakob, 37603, Holzminden (DE); Gatfield, Ian, 37671, Höxter (DE)
(74) Vertreter: Eisenführ, Speiser & Partner

(56) Entgegenhaltungen:
- ES-A1- 459 665
- US-A- 4 105 038

## Beschreibung

Die vorliegende Erfindung betrifft ein Verfahren zum Freisetzen bestimmter Flavanone und Dihydrochalkone aus Flavanonglycosiden bzw. Dihydrochalkonglycosiden durch saure Hydrolyse.

Insbesondere betrifft die vorliegende Erfindung ein Verfahren zum Freisetzen von (a) Hesperetin aus Hesperidin oder (b) Phloretin aus Phloridzin durch saure Hydrolyse.

Die nachfolgenden Ausführungen beziehen sich - beispielhaft - überwiegend auf die Freisetzung von (a) Hesperetin aus Hesperidin oder (b) Phloretin aus Phloridzin durch saure Hydrolyse. Neben diesen besonders relevanten Freisetzungen betrifft die vorliegende Erfindung aber allgemeiner die Freisetzung bestimmter Flavanone und Dihydrochalkone aus bestimmten Flavanonglycosiden bzw. Dihydrochalkonglycosiden durch saure Hydrolyse.

Die bestimmten Flavanone im Sinne der Erfindung sind Hesperetin (bevorzugt, siehe oben), Homoeriodictyol und Eriodictyol.

Die bestimmten Flavanonglycoside im Sinne der Erfindung sind Hesperidin (bevorzugt, siehe oben), Neohesperidin, Eriocitrin, Hesperetin-7-O-beta-D-glucosid, Eriodictyol-7-O-beta-D-glucosid, Homoeriodictyol-7-O-beta-D-glucosid, Homoeriodictyol-7,4'-di-O-beta-D-glucosid und Eriodictyol-7,4'-di-O-beta-D-glucosid.
Die bestimmten Dihydrochalkone im Sinne der Erfindung sind Phloretin (bevorzugt, siehe oben), 3-Hydroxyphloretin und Davidigenin.
Die bestimmten Dihydrochalkonglycoside im Sinne der Erfindung sind Phloridzin (bevorzugt, siehe oben), 3-Hydroxyphloretin-2'-O-beta-D-glucosid, Davidigenin-2-O-beta-D-glucosid und Phloretin-2'-(beta-D-xylosyl-(1-6)-beta-D-glucosid).

Hinweise auf die Freisetzung von (a) Hesperetin aus Hesperidin oder (b) Phloretin aus Phloridzin durch saure Hydrolyse gelten - mit den notwendigen Anpassungen - für die anderen genannten Verbindungen.

Verfahren zur synthetischen Herstellung von Flavonoiden wie beispielsweise Hesperetin und Phloretin aber auch zum Beispiel Quercetin aus den entsprechenden Glycosiden durch Säurehydrolyse sind bereits bekannt.

ES 459665 beschreibt die Hydrolyse von Hesperidin durch Erhitzen unter Rückfluss in einem Gemisch aus einem niedermolekularen Alkohol (1-3 C-Atome) und 3 N Salzsäure oder Schwefelsäure für mindestens zwei Stunden.

US 4,150,038 (Wingard) beschreibt die Freisetzung von Hesperetin aus Hesperidin durch Erhitzen unter Rückfluss in einem Gemisch aus starker, nicht oxidierender Mineralsäure und niedermolekularen, primären Alkanol. Der Alkanol umfasst nicht mehr als 10 Gew.-% Wasser. Es wird darauf hingewiesen, dass phosphorhaltige Mineralsäuren, schwefelige Säure und organische Säuren zu schwach seien, um die gewünschte Reaktion effektiv zu katalysieren.

J. Org. Chem. 25, 1829 (1960) (Looker et al) offenbart ein Verfahren zur Freisetzung von Hesperetin aus Hesperidin, in dem das Glycosid für 60 bis 72 Stunden in 2%iger Schwefelsäure in 50 % Ethanol erhitzt wird.

Carbohydrate Research 328 (2000) 141-146 (Grohmann et al) offenbart die Hydrolyse einer wässrigen Hesperidin-Suspension mit verdünnter Schwefelsäure bei Temperaturen im Bereich von 100 bis 180 °C. Der Schwerpunkt der beschriebenen Untersuchungen liegt auf der partiellen Hydrolyse des Hesperidins unter Bildung des Hesperetin-7-Glucosids.

Andere Verfahren zur Freisetzung von Hesperetin aus Hesperidin, die nicht auf einer sauren Hydrolyse basieren, ergeben sich beispielsweise aus Veröffentlichungen von Kim et al. (Kim, Youn Chul; Higuchi, Ryuichi; Kitamura, Yoichi; Komori, Tetsuya, Thermal degradation of glycosides. IV. Degradation of flavonoid glycosides, Liebigs Annalen der Chemie (1991), 12, 1285-1289) sowie Miyake et al. (Miyake, Yoshiaki; Minato, Kenichiro; Fukumoto, Syuichi; Yamamoto, Kanefumi; Oya-Ito, Tomoko; Kawakishi, Syunro; Osawa, Toshihiko; New potent antioxidative hydroxyflavanones produced with Aspergillus saitoi from flavanone glycoside in citrus fruit, Biosci. Biotechnol. Biochem. (2003), 67 (7), 1443 - 1450).

Trotz der vorstehend genannten und anderer Verfahren zur Freisetzung von Hesperetin aus Hesperidin (und entsprechend zur Freisetzung von Phloretin aus Phloridzin) besteht weiterhin ein Bedarf an verbesserten Verfahren zur Freisetzung der besagten Flavonoide aus den entsprechenden Glycosiden. Die bisher bekannten Verfahren besitzen nämlich jeweils einen oder mehrere der nachfolgenden Nachteile:

Geringe Produktivität (geringe Eduktkonzentration, niedrige Ausbeute, lange Reaktionszeit); geringe Reinheit; Entstehung unerwünschter Nebenprodukte (z.B. Zersetzungsprodukte der durch die Hydrolyse freigesetzten Zucker (Rhamnose; Glucose).

Hesperetin und Phloretin besitzen positive Eigenschaften im Rahmen einer gesunden Ernährung. Auch daher besteht ein starkes Interesse an technischen Produktionsverfahren, bei denen die besagten Flavonoide in hoher Ausbeute und Reinheit unter natürlichen Bedingungen hergestellt werden können.

Es war daher die Aufgabe der vorliegenden Erfindung, ein Verfahren - allgemein - zur Freisetzung bestimmter Flavanone und Dihydrochalkone aus Flavanonglycosiden bzw. Dihydrochalkonglycosiden und - speziell - zur Freisetzung von Hesperetin aus Hesperidin bzw. zur Freisetzung von Phloretin aus Phloridzin anzugeben, das bei Einsatz hoher Eduktkonzentrationen zu hohen Ausbeuten führt. Vorzugsweise sollte es in dem anzugebenden Verfahren möglich sein, auch die jeweiligen freigesetzten Zucker zu gewinnen; dies trifft insbesondere auf die Rhamnose zu, welche aus Hesperidin freigesetzt wird. Die gestellte allgemeine Aufgabe wird gelöst durch ein Verfahren zum Freisetzen von
(a) Hesperetin, Homoeriodictyol oder Eriodictyol aus Hesperidin, Neohesperidin, Eriocitrin, Hesperetin-7-O-beta-D-glucosid, Eriodictyol-7-O-beta-D-glucosid, Homoeriodictyol-7-O-beta-D-glucosid, Homoeriodictyol-7,4'-di-O-beta-D-glucosid bzw. Eriodictyol-7,4'-di-O-beta-D-glucosid oder
(b) Phloretin, 3-Hydroxyphloretin oder Davidigenin aus Phloridzin, 3-Hydroxyphloretin-2'-O-beta-D-glucosid, Davidigenin-2-O-beta-D-glucosid bzw. Phloretin-2'-(beta-D-xylosyl-(1-6)-beta-D-glucosid)
durch saure Hydrolyse, mit folgenden Schritten:
(i) Suspendieren oder Lösen von (a) Hesperidin, Neohesperidin, Eriocitrin, Hesperetin-7-O-beta-D-glucosid, Eriodictyol-7-O-beta-D-glucosid, Homoeriodictyol-7-O-beta-D-glucosid, Homoeriodictyol-7,4'-di-O-beta-D-glucosid bzw. Eriodictyol-7,4'-di-O-beta-D-glucosid bzw. (b) Phloridzin, 3-Hydroxyphloretin-2'-O-beta-D-glucosid, Davidigenin-2-O-beta-D-glucosid bzw. Phloretin-2'-(beta-D-xylosyl-(1-6)-beta-D-glucosid) in einem Lösungs- oder Suspensionsmittel umfassend oder bestehend aus einer die saure Hydrolyse katalysierenden Menge einer organischen Säure und Wasser;
(ii) Erhitzen der resultierenden Mischung auf eine Temperatur im Bereich von 100 bis 160 °C und Halten bei dieser Temperatur für einen Zeitraum im Bereich von 1 bis 12 Stunden;
(iii) Abtrennen des (a) Hesperetins, Homoeriodictyols bzw. Eriodictyols bzw. (b) Phloretins, 3-Hydroxyphloretins bzw. Davidigenins aus der resultierenden Produktmischung.

Eigene Untersuchungen gingen zunächst - ähnlich wie die oben angegebenen Veröffentlichungen - in unterschiedliche technische Richtungen. Angesichts der oben referierten Annahme überraschend, dass organische Säuren zur effektiven Katalyse der Hydrolyse von Hesperidin nicht geeignet seien, stellte sich dann jedoch heraus, dass ein - spezielles - Verfahren zum Freisetzen von (a) Hesperetin aus Hesperidin bzw. (b) Phloretin aus Phloridzin durch saure Hydrolyse in besonderer Weise geeignet ist, die gestellte Aufgabe zu lösen, sofern es die folgenden Schritte umfasst:
(i) Suspendieren oder Lösen von (a) Hesperidin bzw. (b) Phloridzin in einem Lösungs- oder Suspensionsmittel umfassend oder bestehend aus einer die saure Hydrolyse katalysierenden Menge einer organischen Säure und Wasser;
(ii) Erhitzen der resultierenden Mischung auf eine Temperatur im Bereich von 100 bis 160 °C und Halten bei dieser Temperatur für einen Zeitraum im Bereich von 1 bis 12 Stunden;
(iii) Abtrennen des (a) Hesperetins bzw. (b) Phloretins aus der resultierenden Produktmischung.

Überraschenderweise erwiesen sich die erfindungsgemäßen einzusetzenden organischen Säuren sehr wohl als hervorragende Hydrolysekatalysatoren. Gleichzeitig werden durch die Verfahrensparameter in den Schritten (i) und (ii) insgesamt Bedingungen geschaffen, bei denen Hesperetin bzw. Phloretin in hohen Ausbeuten erhalten werden können. Die bei der Hydrolyse anfallenden Zucker, insbesondere die bei Hydrolyse von Hesperidin anfallende Rhamnose können aus dem Produktgemisch isoliert werden, ohne dass es üblicherweise unter den Reaktionsbedingungen zu einer nennenswerten Zersetzung der Zuckermoleküle kommt. Somit resultiert aus der Hydrolyse von z.B. Hesperidin nicht nur ein einzelner Wertstoff (Hesperetin), sondern sogleich zwei (Hesperetin; Rhamnose).

Vorzugsweise umfasst ein erfindungsgemäßes Verfahren die folgenden Schritte:
(i) Suspendieren oder Lösen von 5 bis 50 Gew.-%, bevorzugt 10 bis 30 Gew.-%, (a) Hesperidin bzw. (b) Phloridzin in einem Lösungs- oder Suspensionsmittel umfassend:
   - 5 bis 50 Gew.-% Aceton, Methanol, Ethanol, n-Propanol, i-Propanol, n-Butanol oder eine Mischung dieser Verbindungen und
   - Rest Wasser sowie eine die saure Hydrolyse katalysierende Menge einer organischen Säure,
   wobei die Gewichtsprozentangaben jeweils bezogen sind auf die Gesamtmenge der Lösung oder Suspension;
(ii) Erhitzen der resultierenden Mischung auf eine Temperatur im Bereich von 100 bis 160 °C und Halten bei dieser Temperatur für einen Zeitraum im Bereich von 1 bis 12 Stunden,
(iii) Ausfällen des (a) Hesperetins bzw. (b) Phloretins aus der resultierenden Produktmischung.

Vorzugsweise wird das erfindungsgemäße Verfahren so ausgestaltet, dass die nach Schritt (i) vorliegende Suspension oder Lösung neben der die saure Hydrolyse katalysierenden Menge der organischen Säure umfasst:
- 5 bis 30 Gew.-% (a) Hesperidin bzw. (b) Phloridzin,
- 10 bis 40 Gew.-% insgesamt an Aceton und Ethanol,
- 30 bis 85 Gew.-% Wasser,

bezogen auf die Gesamtmenge der Suspension oder Lösung.

Gemäß den vorstehend erörterten bevorzugten Ausgestaltungen eines erfindungsgemäßen Verfahrens wird in Schritt (i) ein organisches Lösungs- oder Suspensionsmittel eingesetzt, bei dem es sich vorzugsweise um Aceton, Methanol, Ethanol, n-Propanol, i-Propanol, n-Butanol oder eine Mischung dieser Verbindungen handelt.

In erfindungsgemäßen Verfahren ist die organische Säure vorzugsweise ausgewählt aus der Gruppe organischer Säuren mit mindestens 2 C-Atomen und deren Mischungen. Besonders bevorzugt ist die organische Säure ausgewählt aus der Gruppe bestehend aus: Zitronensäure, Oxalsäure, Milchsäure, Essigsäure und deren Mischungen.

Alternativ zum Einsatz der oben genannten organischen Lösungs- oder Suspensionsmittel kann in Schritt (i) eines erfindungsgemäßen Verfahrens das Lösungs-oder Suspensionsmittel so gewählt sein, dass es Milchsäure, Wasser und gegebenenfalls Lactid umfasst oder daraus besteht, wobei die Menge an Wasser zumindest equimolar zu der eingesetzten Menge an- allgemein -
(a) Hesperidin, Neohesperidin, Eriocitrin, Hesperetin-7-O-beta-D-glucosid, Eriodictyol-7-O-beta-D-glucosid, Homoeriodictyol-7-O-beta-D-glucosid, Homoeriodictyol-7,4'-di-O-beta-D-glucosid bzw. Eriodictyol-7,4'-di-O-beta-D-glucosid oder
(b) Phloridzin, 3-Hydroxyphloretin-2'-O-beta-D-glucosid, Davidigenin-2-O-beta-D-glucosid bzw. Phloretin-2'-(beta-D-xylosyl-(1-6)-beta-D-glucosid) ist.

Speziell - bezogen auf die bevorzugten Produkte - ist die Menge an Wasser zumindest equimolar zu der eingesetzten Menge an (a) Hesperidin bzw. (b) Phloridzin.

Technische Milchsäure umfasst eine recht hohe Menge an Wasser und kann deshalb direkt, d. h. ohne Wasserzusatz als Lösungs- oder Suspensionsmittel eingesetzt werden.

Bei Einsatz von Milchsäure und Wasser als Lösungs- oder Suspensionsmittel umfasst die nach Schritt (i) des erfindungsgemäßen Verfahrens vorliegende Lösung oder Suspension vorzugsweise 5 bis 50 Gew.-%, bevorzugt 10 bis 30 Gew.-%, (a) Hesperidin bzw. (b) Phloridzin, bezogen auf die Gesamtmenge der Suspension oder Lösung.

Vorteilhafterweise wird in einem erfindungsgemäßen Verfahren Hesperidin hydrolysiert und aus der entsprechenden Produktmischung sowohl in einem entsprechenden Schritt Hesperetin als auch in einem zusätzlichen Schritt Rhamnose abgetrennt.

Die abgetrennte Rhamnose (CAS 10030-85-0) ist ein wertvoller kosmetischer Wirkstoff und kann beispielsweise verwendet werden für die Synthese von Furanonen wie 2,5-Dimethyl-4-hydroxy-3(2H)-furanon (Furaneol). Allgemein besitzt Rhamnose Bedeutung für enzymatische und biochemische Untersuchungen als Nährmedium sowie als Synthesebaustein; Rhamnose hat als Vorstufe für Reaktionsaromen und als chiraler Baustein in der chemischen Synthese Bedeutung.

Unabhängig von der weiteren Ausgestaltung eines erfindungsgemäßen Verfahrens sollte die nach Schritt (i) vorliegende Suspension oder Lösung einen pH kleiner 4, vorzugsweise kleiner 3 besitzen.

Entsprechend sollte in Schritt (ii) die resultierende Mischung vorzugsweise auf eine Temperatur im Bereich von 125 bis 145 °C erhitzt und bei dieser Temperatur für einen Zeitraum im Bereich von 1 bis 12 Stunden gehalten werden.

Durch das erfindungsgemäße Verfahren erhält man die Flavanone Hesperetin (bevorzugt, siehe oben), Homoeriodictyol und Eriodictyol als (2S)- oder (2R)-Enantiomere oder als beliebige Mischung der Enantiomere. Die Chiralität des Flavanon-Teils der Flavanonglycoside wird dabei weitgehend erhalten.

Das erfindungsgemäße Verfahren ist geeignet zur Herstellung von Reaktionsaromen, d.h. von Aromen die entstehen, wenn eine geeignete Mischung von Ausgangserzeugnissen zu diesem Zweck erhitzt wird.

Die vorliegende Erfindung betrifft auch die Verwendung einer organischen Säure als Hydrolysekatalysator beim Freisetzen von
(a) Hesperetin, Homoeriodictyol oder Eriodictyol aus Hesperidin, Neohesperidin, Eriocitrin, Hesperetin-7-O-beta-D-glucosid, Eriodictyol-7-O-beta-D-glucosid, Homoeriodictyol-7-O-beta-D-glucosid, Homoeriodictyol-7,4'-di-O-beta-D-glucosid bzw. Eriodictyol-7,4'-di-O-beta-D-glucosid oder
(b) Phloretin, 3-Hydroxyphloretin oder Davidigenin aus Phloridzin, 3-Hydroxyphloretin-2'-O-beta-D-glucosid, Davidigenin-2-O-beta-D-glucosid bzw. Phloretin-2'-(beta-D-xylosyl-(1-6)-beta-D-glucosid)
in industriellem Maßstab.

Insbesondere betrifft die Erfindung die Verwendung einer organischen Säure als Hydrolysekatalysator beim Freisetzen von Verwendung einer organischen Säure als Hydrolysekatalysator beim Freisetzen von
(a) Hesperetin aus Hesperidin oder
(b) Phloretin aus Phloridzin
in industriellem Maßstab.

"Industrieller Maßstab" bedeutet hierbei, dass die nach Schritt (i) vorliegende Mischung (Suspension oder Lösung) ein Volumen von zumindest 100 Litern besitzt.

Weitere Aspekte der vorliegenden Erfindung ergeben sich aus den Beispielen und den beigefügten Patentansprüchen.

Nachfolgend wird die Erfindung anhand von Beispielen näher erläutert:

### Beispiel 1: Freisetzung von Hesperetin aus Hesperidin

### Reaktion:

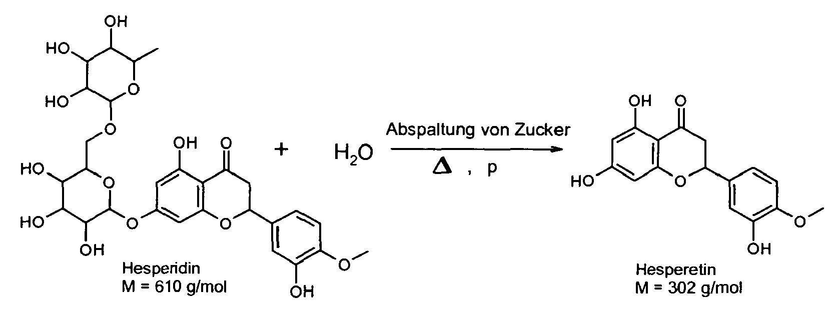

### Einsatzstoffe:

Pos. Menge Stoffmenge

| | [kg] | [mol] | Name |
|---|---|---|---|
| | | | |
| 1) | 10,0 | 16,4 | Hesperidin |
| 2) | 22,5 | 388 | Aceton |
| 3) | 66,2 | 3678 | Wasser, städt. |
| 4) | 1,3 | 6,6 | Citronensäure |
| 5) | 0,096 | 0,27 | Na₂HPO₄ x 12 H₂O |
| 6) | 95 | 5278 | Wasser, städt. |
| | 195,096 | | |

### Durchführung:

- Pos. 4) und 5) im Wasser (Pos.3) lösen,
- Pos. 2) vorlegen
- Pos. 1) unter Rühren im Aceton (Pos. 2) suspendieren
- das wässrige Gemisch aus Pos. 3)-5) zu der Suspension aus Pos. 1)-2) zugeben
- das resultierende Gemisch in den Reaktor überführen
- Gemisch unter Rühren auf 145 °C aufheizen, dabei erhöht sich der Druck auf ca. 7,5 bar
- 8 Std. bei 145°C rühren
- das Gemisch auf 50°C abkühlen lassen und mit Pos. 6) versetzen, um das Produkt auszufällen
- das Produktgemisch auf die Drucknutsche ablassen und die Flüssigkeit abdrücken, waschen und anschließend trocknen

### Auswertung:

Der getrocknete und homogenisierte Feststoff wurde per HPLC analysiert. Typischerweise wurde eine Reinheit > 90 % erreicht.

### Ausbeute: ca. 84 %, Reinheit: 90 %

### Hinweis:

Die in wässriger Lösung vorliegende Rhamnose (als weiteres Produkt der sauren Hydrolyse) kann (ebenso wie die parallel vorliegende Glucose) abgetrennt werden, z.B. durch Eindicken der Lösung und anschließendes Auskristallisieren.

### Beispiel 2: Freisetzung von Hesperetin aus Hesperidin

Vorgehensweise analog Beispiel 1, jedoch wird anstelle von Aceton Ethanol als Lösungsmittel/Suspensionsmittel eingesetzt.

Ausbeute und Reinheit entsprachen der gemäß Beispiel 1.

### Beispiel 3: Freisetzen von Hesperetin aus Hesperidin unter Verwendung technischer Milchsäure:

20 g Hesperidin wurden in 80 g technischer Milchsäure (Wasseranteil ca. 20 %) suspendiert. Das Gemisch wurde unter Rühren und unter Rückfluss erhitzt (ca. 126 °C). Nach ca. 4 h war die Hydrolyse des Hesperidins nahezu vollständig. Die Analyse erfolgte per HPLC:

### Ergebnis:

| **Probe** | **Hesperidin** | **Hesperetin** |
|---|---|---|
| vor Erhitzen | 20 % | n. n. |
| nach Erhitzen (ca. 4 h) | n. n. | 5,9 Gew.-% |

| | | |
|---|---|---|
| n. n. = nicht nachweisbar | | |

### Beispiel 4: Freisetzung von Phloretin aus Phloridzin:

Die Vorgehensweise entsprach aus der aus Beispiel 1, wobei lediglich anstelle des dort eingesetzten Hesperidins Phloridzin eingesetzt wurde. Ausbeute und Reinheit entsprachen den Ergebnissen aus Beispiel 1.

### Beispiel 5: Freisetzen von Phloretin aus Phloridzin, Einsatz eines Phloridzin-haltigen Apfelsaftes:

50 g einer polyphenolreichen Apfelsaftfraktion (ca. 0,3 Gew.-% Phloridzin) wurden mit 50 g Ethanol und 50 g einer wässrigen Zitronensäurelösung (0,1 M) versetzt und im Autoklaven bei 145 °C für 4 Stunden erhitzt. Im Anschluss an die Hydrolyse wurde per HPLC analysiert:

| **Probe** | **Phloridzin** | **Phloretin** |
|---|---|---|
| vor Erhitzen | 925 ppm | n. n. |
| nach Erhitzen (ca. 4 h) | 150 ppm | 650 ppm |

| | | |
|---|---|---|
| n. n. = nicht nachweisbar | | |

### Beispiel 6: Herstellung eines Reaktionsaromas und Verkostung

Es wurden die folgenden Substanzen A bis F in den angegebenen Mengen bereitgestellt.

| | | | |
|---|---|---|---|
| A: | 20 | g | Hesperidin |
| B: | 40 | g | Milchsäure |
| | | | |
| C: | 40 | g | Di - Ethyl - L Tartrat |
| | | | |
| D: | 0,6 | g | Dextrose |
| | | | |
| E: | 0,6 | g | Alanin |
| | | | |
| F: | 0,4 | g | Vanillin |

Es wurde gemäß der folgenden Vorschrift ein Reaktionsaroma hergestellt:
Die Positionen A bis F werden gemischt und 6,5 Stunden unter Rückfluss gekocht.

Nach durchgeführter Reaktion wurde der Gehalt an bestimmten Inhaltsstoffen in der Produktmischung bestimmt, vgl. die nachfolgende Tabelle:

| | Gehalt Hesperidin | Gehalt Hesperetin-Monoglucosid | Gehalt Hesperetin |
|---|---|---|---|
| nach Reaktion | 4,30% | 1,10% | 5,10% |

Das Reaktionsprodukt wurde gemäß der folgenden Vorschrift verkostet:

Zur Verkostung wird das Reaktionsprodukt zunächst in einem Lösemittel (z.B. Propylenglycol) gelöst, so dass ein Hesperetin-Gehalt von ca. 2 % erhalten wird. Die Verkostung wird dann in der Weise durchgeführt, dass eine Konzentration von ca. 50 ppm an Hesperetin in einer 5 %igen Zuckerlösung angesetzt wird.

Anmerkung: Im Vergleich zur Hydrolyse in Milchsäure bei Abwesenheit von Diethyltartrat ist die vorstehend geschilderte Verfahrensgestaltung, bei der die Hydrolyse in Anwesenheit von Milchsäure und Diethyltartrat stattfindet, deutlich weniger sauer und es kommt daher zu einer verbesserten Süßewahrnehmung.

## Patentansprüche

1. Verfahren zum Freisetzen von
(a) Hesperetin, Homoeriodictyol oder Eriodictyol aus Hesperidin, Neohesperidin, Eriocitrin, Hesperetin-7-O-beta-D-glucosid, Eriodictyol-7-O-beta-D-glucosid, Homoeriodictyol-7-O-beta-D-glucosid, Homoeriodictyol-7,4'-di-O-beta-D-glucosid bzw. Eriodictyol-7,4'-di-O-beta-D-glucosid oder
(b) Phloretin, 3-Hydroxyphloretin oder Davidigenin aus Phloridzin, 3-Hydroxyphloretin-2'-O-beta-D-glucosid, Davidigenin-2-O-beta-D-glucosid bzw. Phloretin-2'-(beta-D-xylosyl-(1-6)-beta-D-glucosid)
durch saure Hydrolyse, mit folgenden Schritten:
(i) Suspendieren oder Lösen von (a) Hesperidin, Neohesperidin, Eriocitrin, Hesperetin-7-O-beta-D-glucosid, Eriodictyol-7-O-beta-D-glucosid, Homoeriodictyol-7-O-beta-D-glucosid, Homoeriodictyol-7,4'-di-O-beta-D-glucosid bzw. Eriodictyol-7,4'-di-O-beta-D-glucosid bzw. (b) Phloridzin, 3-Hydroxyphloretin-2'-O-beta-D-glucosid, Davidigenin-2-O-beta-D-glucosid bzw. Phloretin-2'-(beta-D-xylosyl-(1-6)-beta-D-glucosid) in einem Lösungs- oder Suspensionsmittel umfassend oder bestehend aus einer die saure Hydrolyse katalysierenden Menge einer organischen Säure und Wasser;
(ii) Erhitzen der resultierenden Mischung auf eine Temperatur im Bereich von 100 bis 160 °C und Halten bei dieser Temperatur für einen Zeitraum im Bereich von 1 bis 12 Stunden;
(iii) Abtrennen des (a) Hesperetins, Homoeriodictyols bzw. Eriodictyols bzw. (b) Phloretins, 3-Hydroxyphloretins bzw. Davidigenins aus der resultierenden Produktmischung.

2. Verfahren nach Anspruch 1 zum Freisetzen von
(a) Hesperetin aus Hesperidin oder
(b) Phloretin aus Phloridzin
durch saure Hydrolyse, mit folgenden Schritten:
(i) Suspendieren oder Lösen von (a) Hesperidin bzw. (b) Phloridzin in einem Lösungs- oder Suspensionsmittel umfassend oder bestehend aus einer die saure Hydrolyse katalysierenden Menge einer organischen Säure und Wasser;
(ii) Erhitzen der resultierenden Mischung auf eine Temperatur im Bereich von 100 bis 160 °C und Halten bei dieser Temperatur für einen Zeitraum im Bereich von 1 bis 12 Stunden;
(iii) Abtrennen des (a) Hesperetins bzw. (b) Phloretins aus der resultierenden Produktmischung.

3. Verfahren nach Anspruch 2, mit folgenden Schritten:
(i) Suspendieren oder Lösen von 5 bis 50 Gew.-%, bevorzugt 10 bis 30 Gew.-%, (a) Hesperidin bzw. (b) Phloridzin in einem Lösungs- oder Suspensionsmittel umfassend:
- 5 bis 50 Gew.-% Aceton, Methanol, Ethanol, n-Propanol, i-Propanol, n-Butanol oder eine Mischung dieser Verbindungen und
- Rest Wasser sowie eine die saure Hydrolyse katalysierende Menge einer organischen Säure,
wobei die Gewichtsprozentangaben jeweils bezogen sind auf die Gesamtmenge der Lösung oder Suspension;
(ii) Erhitzen der resultierenden Mischung auf eine Temperatur im Bereich von 100 bis 160 °C und Halten bei dieser Temperatur für einen Zeitraum im Bereich von 1 bis 12 Stunden,
(iii) Ausfällen des (a) Hesperetins bzw. (b) Phloretins aus der resultierenden Produktmischung.

4. Verfahren nach einem der vorangehenden Ansprüche, wobei die nach Schritt (i) vorliegende Suspension oder Lösung umfasst:
- 5 bis 30 Gew.-% (a) Hesperidin bzw. (b) Phloridzin,
- 10 bis 40 Gew.-% insgesamt an Aceton und Ethanol,
- 30 bis 85 Gew.-% Wasser,
bezogen auf die Gesamtmenge der Suspension oder Lösung.

5. Verfahren nach einem der vorangehenden Ansprüche, wobei die organische Säure ausgewählt ist aus der Gruppe organischer Säuren mit mindestens 2 C-Atomen und deren Mischungen und vorzugsweise ausgewählt ist aus der Gruppe bestehend aus: Zitronensäure, Oxalsäure, Milchsäure, Essigsäure und deren Mischungen.

6. Verfahren nach Anspruch 1 oder 2, wobei in Schritt (i) das Lösungs- oder Suspensionsmittel eine Mischung umfassend oder bestehend aus Milchsäure, Wasser und gegebenenfalls Lactid ist, wobei die Menge an Wasser zumindest equimolar zu der eingesetzten Menge an
(a) Hesperidin, Neohesperidin, Eriocitrin, Hesperetin-7-O-beta-D-glucosid, Eriodictyol-7-O-beta-D-glucosid, Homoeriodictyol-7-O-beta-D-glucosid, Homoeriodictyol-7,4'-di-O-beta-D-glucosid bzw. Eriodictyol-7,4'-di-O-beta-D-glucosid oder
(b) Phloridzin, 3-Hydroxyphloretin-2'-O-beta-D-glucosid, Davidigenin-2-O-beta-D-glucosid bzw. Phloretin-2'-(beta-D-xylosyl-(1-6)-beta-D-glucosid) ist.

7. Verfahren nach Anspruch 6, wobei die nach Schritt (i) vorliegende Lösung oder Suspension 5 bis 50 Gew.-%, bevorzugt 10 bis 30 Gew.-%, (a) Hesperidin bzw. (b) Phloridzin umfasst, bezogen auf die Gesamtmenge der Suspension oder Lösung.

8. Verfahren zum Freisetzen von Hesperetin aus Hesperidin nach einem der vorangehenden Ansprüche, wobei aus der Produktmischung Hesperetin abgetrennt sowie in einem zusätzlichen Schritt Rhamnose abgetrennt wird.

9. Verfahren nach einem der vorangehenden Ansprüche, wobei die nach Schritt (i) vorliegende Suspension oder Lösung einen pH kleiner 4, vorzugsweise kleiner 3 besitzt.

10. Verfahren nach einem der vorangehenden Ansprüche, wobei in Schritt (ii) die resultierende Mischung auf eine Temperatur im Bereich von 125 bis 145 °C erhitzt und bei dieser Temperatur für einen Zeitraum im Bereich von 1 bis 12 Stunden gehalten wird.

11. Verwendung einer organischen Säure als Hydrolysekatalysator beim Freisetzen von
(a) Hesperetin, Homoeriodictyol oder Eriodictyol aus Hesperidin, Neohesperidin, Eriocitrin, Hesperetin-7-O-beta-D-glucosid, Eriodictyol-7-O-beta-D-glucosid, Homoeriodictyol-7-O-beta-D-glucosid, Homoeriodictyol-7,4'-di-O-beta-D-glucosid bzw. Eriodictyol-7,4'-di-O-beta-D-glucosid oder
(b) Phloretin, 3-Hydroxyphloretin oder Davidigenin aus Phloridzin, 3-Hydroxyphloretin-2'-O-beta-D-glucosid, Davidigenin-2-O-beta-D-glucosid bzw. Phloretin-2'-(beta-D-xylosyl-(1-6)-beta-D-glucosid)
in industriellem Maßstab.

12. Verwendung einer organischen Säure als Hydrolysekatalysator beim Freisetzen von
(a) Hesperetin aus Hesperidin oder
(b) Phloretin aus Phloridzin
in industriellem Maßstab.

## Claims

1. Method for the release of
(a) hesperetin, homoeriodictyol or eriodictyol from hesperidin, neohesperidin, eriocitrin, hesperetin 7-O-beta-D-glucoside, eriodictyol 7-O-beta-D-glucoside, homoeriodictyol 7-O-beta-D-glucoside, homoeriodictyol 7,4'-di-O-beta-D-glucoside or eriodictyol 7,4'-di-O-beta-D-glucoside or
(b) phloretin, 3-hydroxyphloretin or davidigenin from phloridzin, 3-hydroxyphloretin 2'-O-beta-D-glucoside, davidigenin 2-O-beta-D-glucoside or phloretin 2'-(beta-D-xylosyl-(1-6)-beta-D-glucoside)
by acid hydrolysis, with the following steps:
(i) suspending or dissolving (a) hesperidin, neohesperidin, eriocitrin, hesperetin 7-O-beta-D-glucoside, eriodictyol 7-O-beta-D-glucoside, homoeriodictyol 7-O-beta-D-glucoside, homoeriodictyol 7,4'-di-O-beta-D-glucoside or eriodictyol 7,4'-di-O-beta-D-glucoside or (b) phloridzin, 3-hydroxyphloretin 2'-O-beta-D-glucoside, davidigenin 2-O-beta-D-glucoside or phloretin 2'-(beta-D-xylosyl-(1-6)-beta-D-glucoside) in a solvent or suspending agent comprising or consisting of an organic acid in an amount which catalyses the acid hydrolysis and water;
(ii) heating the resulting mixture to a temperature in the range of from 100 to 160 °C and keeping it at this temperature for a period in the range of from 1 to 12 hours;
(iii) separating off the (a) hesperetin, homoeriodictyol or eriodictyol or (b) phloretin, 3-hydroxyphloretin or davidigenin from the resulting product mixture.

2. Method according to claim 1, for the release of
(a) hesperetin from hesperidin or
(b) phloretin from phloridzin
by acid hydrolysis, with the following steps:
(i) suspending or dissolving (a) hesperidin or (b) phloridzin in a solvent or suspending agent comprising or consisting of an organic acid in an amount which catalyses the acid hydrolysis and water;
(ii) heating the resulting mixture to a temperature in the range of from 100 to 160 °C and keeping it at this temperature for a period in the range of from 1 to 12 hours;
(iii) separating off the (a) hesperetin or (b) phloretin from the resulting product mixture.

3. Method according to claim 2, with the following steps:
(i) suspending or dissolving 5 to 50 wt.%, preferably 10 to 30 wt.% of (a) hesperidin or (b) phloridzin in a solvent or suspending agent comprising:
- 5 to 50 wt.% of acetone, methanol, ethanol, n-propanol, i-propanol, n-butanol or a mixture of these compounds and
- the remainder as water and an organic acid in an amount which catalyses the acid hydrolysis, the per cent by weight data in each case being based on the total amount of the solution or suspension;
(ii) heating the resulting mixture to a temperature in the range of from 100 to 160 °C and keeping it at the temperature for a period in the range of from 1 to 12 hours,
(iii) precipitating the (a) hesperetin or (b) phloretin from the resulting product mixture.

4. Method according to one of the preceding claims, wherein the suspension or solution present after step (i) comprises:
- 5 to 30 wt.% of (a) hesperidin or (b) phloridzin,
- 10 to 40 wt.% in total of acetone and ethanol,
- 30 to 85 wt.% of water,
based on the total amount of the suspension or solution.

5. Method according to one of the preceding claims, wherein the organic acid is chosen from the group of organic acids having at least 2 C atoms and mixtures thereof, and is preferably chosen from the group consisting of: citric acid, oxalic acid, lactic acid, acetic acid and mixtures thereof.

6. Method according to claim 1 or 2, wherein in step (i) the solvent or suspending agent is a mixture comprising or consisting of lactic acid, water and optionally lactide, the amount of water being at least equimolar to the amount of
(a) hesperidin, neohesperidin, eriocitrin, hesperetin 7-O-beta-D-glucoside, eriodictyol 7-O-beta-D-glucoside, homoeriodictyol 7-O-beta-D-glucoside, homoeriodictyol 7,4'-di-O-beta-D-glucoside or eriodictyol 7,4'-di-O-beta-D-glucoside or
(b) phloridzin, 3-hydroxyphloretin 2'-O-beta-D-glucoside, davidigenin 2-O-beta-D-glucoside or phloretin 2'-(beta-D-xylosyl-(1-6)-beta-D-glucoside)
employed.

7. Method according to claim 6, wherein the solution or suspension present after step (i) comprises 5 to 50 wt.%, preferably 10 to 30 wt.% of (a) hesperidin or (b) phloridzin, based on the total amount of the suspension or solution.

8. Method for the release of hesperetin from hesperidin according to one of the preceding claims, wherein from the product mixture, hesperetin is separated off and in an additional step rhamnose is separated off.

9. Method according to one of the preceding claims, wherein the suspension or solution present after step (i) has a pH of less than 4, preferably less than 3.

10. Method according to one of the preceding claims, wherein in step (ii) the resulting mixture is heated to a temperature in the range of from 125 to 145°C and is kept at this temperature for a period in the range of from 1 to 12 hours.

11. Use of an organic acid as a hydrolysis catalyst in the release of
(a) hesperetin, homoeriodictyol or eriodictyol from hesperidin, neohesperidin, eriocitrin, hesperetin 7-O-beta-D-glucoside, eriodictyol 7-O-beta-D-glucoside, homoeriodictyol 7-O-beta-D-glucoside, homoeriodictyol 7,4'-di-O-beta-D-glucoside or eriodictyol 7,4'-di-O-beta-D-glucoside or
(b) phloretin, 3-hydroxyphloretin or davidigenin from phloridzin, 3-hydroxyphloretin 2'-O-beta-D-glucoside, davidigenin 2-O-beta-D-glucoside or phloretin 2'-(beta-D-xylosyl-(1-6)-beta-D-glucoside)
on an industrial scale.

12. Use of an organic acid as a hydrolysis catalyst in the release of
(a) hesperetin from hesperidin or
(b) phloretin from phloridzin
on an industrial scale.

## Revendications

1. Procédé pour libérer
(a) l'hespérétine, l'homoériodictyol ou l'ériodictyol à partir de l'hespéridine, de la néohespéridine, de l'ériocitrine, de l'hespérétine-7-O-bêta-D-glucoside, de l'ériodictyol-7-O-bêta-D-glucoside, de l'homoériodictyol-7-O-bêta-D-glucoside, de l'homoériodictyol-7,4'-di-O-bêta-D-glucoside ou de l'ériodictyol-7,4'-di-O-bêta-D-glucoside, ou
(b) la phlorétine, la 3-hydroxyphlorétine ou la davidigénine à partir de la phloridzine, du 3-hydroxyphlorétine-2'-O-bêta-D-glucoside, du davidigénine-2-O-bêta-D-glucoside ou du phlorétine-2'-(bêta-D-xylosyl-(1-6)-bêta-D-glucoside)
par hydrolyse acide, comportant les étapes suivantes :
(i) mise en suspension ou dissolution (a) d'hespéridine, de néohespéridine, d'ériocitrine, d'hespérétine-7-O-bêta-D-glucoside, d'ériodictyol-7-O-bêta-D-glucoside, d'homoériodictyol-7-O-bêta-D-glucoside, d'homoériodictyol-7,4'-di-O-bêta-D-glucoside ou d'ériodictyol-7,4'-di-O-bêta-D-glucoside, ou (b) de phloridzine, de 3-hydroxyphlorétine-2'-O-bêta-D-glucoside, de davidigénine-2-O-bêta-D-glucoside ou de phlorétine-2'-(bêta-D-xylosyl-(1-6)-bêta-D-glucoside), dans un solvant ou un agent de mise en suspension comprenant une quantité d'un acide organique catalysant l'hydrolyse acide, et de l'eau, ou qui en est constitué ;
(ii) chauffage du mélange obtenu à une température comprise dans la plage de 100 à 160°C, et maintien à cette température pendant un laps de temps compris dans la plage de 1 à 12 heures ;
(iii) séparation, d'avec le mélange de produits obtenu, (a) de l'hespérétine, de l'homoériodictyol ou de l'ériodictyol, ou (b) de la phlorétine, de la 3-hydroxyphlorétine ou de la davidigénine.

2. Procédé selon la revendication 1 pour libérer
(a) l'hespérétine à partir de l'hespéridine, ou
(b) la phlorétine à partir de la phloridzine,
par hydrolyse acide, comportant les étapes suivantes :
(i) mise en suspension ou dissolution (a) d'hespéridine, ou (b) de phloridzine, dans un solvant ou un agent de mise en suspension comprenant une quantité d'un acide organique catalysant l'hydrolyse acide, et de l'eau, ou qui en est constitué ;
(ii) chauffage du mélange obtenu à une température comprise dans la plage de 100 à 160°C, et maintien à cette température pendant un laps de temps compris dans la plage de 1 à 12 heures ;
(iii) séparation, d'avec le mélange de produits obtenu, (a) de l'hespérétine ou (b) de la phlorétine.

3. Procédé selon la revendication 2, comportant les étapes suivantes :
(i) mise en suspension ou dissolution de 5 à 50 % en poids, de préférence de 10 à 30 % en poids (a) d'hespéridine ou (b) de phloridzine, dans un solvant ou un agent de mise en suspension comprenant :
- 5 à 50 % en poids d'acétone, de méthanol, d'éthanol, de n-propanol, d'i-propanol, de n-butanol ou d'un mélange de ces composés, et
- le reste étant constitué d'eau, ainsi que d'une quantité d'un acide organique catalysant l'hydrolyse acide,
chacune des indications de pourcentage en poids étant rapportée à la quantité totale de la solution ou de la suspension ;
(ii) chauffage du mélange obtenu à une température comprise dans la plage de 100 à 160°C, et maintien à cette température pendant un laps de temps compris dans la plage de 1 à 12 heures,
(iii) précipitation (a) de l'hespérétine, ou (b) de la phlorétine, à partir du mélange de produits obtenu.

4. Procédé selon l'une des revendications précédentes, dans lequel la suspension ou la solution présente après l'étape (i) comprend :
- 5 à 30 % en poids (a) d'hespéridine, ou (b) de phloridzine,
- 10 à 40 % en poids, au total, d'acétone et d'éthanol,
- 30 à 85 % en poids d'eau,
par rapport à la quantité totale de la suspension ou de la solution.

5. Procédé selon l'une des revendications précédentes, dans lequel l'acide organique est choisi dans le groupe des acides organiques ayant au moins 2 atomes de carbone et les mélanges de ceux-ci, et est de préférence choisi dans le groupe consistant en l'acide citrique, l'acide oxalique, l'acide lactique, l'acide acétique et les mélanges de ceux-ci.

6. Procédé selon la revendication 1 ou 2, dans lequel, dans l'étape (i), le solvant ou l'agent de mise en suspension est un mélange comprenant de l'acide lactique, de l'eau et éventuellement du lactide, ou qui en est constitué, la quantité d'eau étant au moins équimolaire par rapport à la quantité utilisée
(a) d'hespéridine, de néohespéridine, d'ériocitrine, d'hespérétine-7-O-bêta-D-glucoside, d'ériodictyol-7-O-bêta-D-glucoside, d'homoériodictyol-7-O-bêta-D-glucoside, d'homoériodictyol-7,4'-di-O-bêta-glucoside ou d'ériodictyol-7-4'-di-O-bêta-D-glucoside, ou
(b) de phloridzine, de 3-hydroxyphlorétine-2'-O-bêta-D-glucoside, de davidigénine-2-O-bêta-D-glucoside ou de phlorétine-2'-(bêta-D-xylosyl-(1-6)-bêta-D-glucoside).

7. Procédé selon la revendication 6, dans lequel la solution ou la suspension présente après l'étape (i) comprend 5 à 50 % en poids, de préférence 10 à 30 % en poids, (a) d'hespéridine ou (b) de phloridzine, par rapport à la quantité totale de la suspension ou de la solution.

8. Procédé pour libérer l'hespérétine à partir d'hespéridine selon l'une quelconque des revendications précédentes, dans lequel on sépare du mélange de produits l'hespérétine et, dans une étape supplémentaire, le rhamnose.

9. Procédé selon l'une quelconque des revendications précédentes, dans lequel la suspension ou la solution présente après l'étape (i) a un pH inférieur à 4, de préférence inférieur à 3.

10. Procédé selon l'une des revendications précédentes, dans lequel, dans l'étape (ii), le mélange obtenu est chauffé à une température comprise dans la plage de 125 à 145°C et est maintenu à cette température pendant un laps de temps compris dans la plage de 1 à 12 heures.

11. Utilisation, à l'échelle industrielle, d'un acide organique en tant que catalyseur d'hydrolyse lors de la libération
(a) d'hespérétine, d'homoériodictyol ou d'ériodictyol à partir d'hespéridine, de néohespéridine, d'ériocitrine, d'hespérétine-7-O-bêta-D-glucoside, d'ériodictyol-7-O-bêta-D-glucoside, d'homoériodictyol-7-O-bêta-D-glucoside, d'homoériodictyol-7,4'-di-O-bêta-D-glucoside ou d'ériodictyol-7,4'-di-O-bêta-D-glucoside, ou
(b) de phlorétine, de 3-hydroxyphlorétine ou de davidigénine à partir de phloridzine, de 3-hydroxyphlorétine-2'-O-bêta-D-glucoside, de davidigénine-2-O-bêta-D-glucoside ou de phlorétine-2'-(bêta-D-xylosyl-(1-6)-bêta-D-glucoside).

12. Utilisation, à l'échelle industrielle, d'un acide organique en tant que catalyseur d'hydrolyse lors de la libération
(a) d'hespérétine à partir d'hespéridine, ou
(b) de phlorétine à partir de phloridzine.
